# EUROPEAN PATENT APPLICATION

(11) **EP 3 505 617 A1**
(43) Date of publication of application: **03.07.2019**
(21) Application number: 19157975.4
(22) Date of filing: 22.07.2013
(51) Int. Cl.: C12N 1/20

(54) **METHOD OF MAKING AGGLOMERATED MICROBIOLOGICAL MEDIA AND COMPOSITIONS THEREOF**

(30) Priority: 07.08.2012 US 201261680449 P; 07.03.2013 US 201361773852 P
(62) Divisional of application: 13744918.7
(71) Applicant: 3M Innovative Properties Company, Saint Paul, MN 55133-3427 (US)
(72) Inventor: LIU, Jie. J., St. Paul, MN 55133-3427 (US); XIA, Wensheng, St. Paul, MN 55133-3427 (US)
(74) Representative: Herzog, Fiesser & Partner Patentanwälte PartG mbB

(57) **Abstract**

A method of making a flowable, agglomerated nutrient medium is provided. The method comprises forming a nutrient medium composition by placing a powdered nutrient into a fluidized bed-type agglomeration chamber, flowing a fluidizing gas through the chamber, and contacting in the agglomeration chamber the powdered nutrient with an aerosol spray of an agglomeration liquid; and collecting the composition from the chamber. The nutrient component facilitates the growth of a microorganism. The composition formed by the method comprises a population of agglomerated nutrient medium particles and, optionally, nonagglomerated powdered nutrient. The population comprises at least about 30 weight percent agglomerated nutrient particles having an effective particle diameter of about 250-400 microns. Compositions, thin film culture devices, and kits comprising the flowable, dried agglomerated nutrient medium are also provided.

## Description

### CROSS-REFERENCE TO RELATED APPLICATIONS

This application claims the benefit of U.S. Provisional Patent Application Nos. 61/680,449, filed on August 7, 2012 and 61/773,852, filed March 7, 2013, which are incorporated herein by reference in their entirety.

### BACKGROUND

Nutrient media formulations have been used to cultivate a number of cell types including animal, plant and microbial cells. Cells cultivated in culture media catabolize available nutrients and produce useful biological substances such as monoclonal antibodies, hormones, growth factors, viruses and the like. Such products have therapeutic applications and, with the advent of recombinant DNA technology, cells can be engineered to produce large quantities of these products. Thus, the ability to cultivate cells in vitro is not only important for the study of cell physiology, but is also necessary for the production of useful substances which may not otherwise be obtained by cost-effective means.

Typical components of microbial culture media may include protein hydrolysates, inorganic salts, vitamins, trace metals, and carbohydrates, the types and amounts of which may vary depending upon the particular requirements of a given species of microorganism. Because these components tend to be more stable in a dehydrated form, they are frequently provided as dry, powdered formulations. The powdered formulations are added to water and, optionally, sterilized before use.

Culture media are typically produced in liquid form or in powdered form. Each of these forms has particular advantages and disadvantages.

For example, liquid culture medium has the advantage that it is provided ready-to-use (unless supplementation with nutrients or other components is necessary), and that the formulations have been optimized for particular cell types. Liquid media have the disadvantages, however, that they may require the addition of supplements (e.g., a vitamin or cofactor, and antibiotic) for optimal performance in cultivating a particular microorganism. Furthermore, most liquid media require some type of sterilization (e.g., autoclaving, filtration), which can be a time-consuming and/or expensive process.

To overcome some of the disadvantages, liquid culture medium can be made in concentrated form; the concentrate may be diluted to working concentrations prior to use. This approach provides the capability of making larger and variable batch sizes than with standard culture media, and the concentrated media formulations or components thereof often have longer shelf-life. U.S. Patent No. 5,474,931 is directed to culture media concentrate technology and is incorporated herein by reference in its entirety. Despite these advantages, however, concentrated liquid media still have the disadvantages of their need for the addition of supplements, and may be difficult to sterilize economically.

As an alternative to liquid media, powdered culture media are often used. This approach has the advantages that larger batch sizes may be produced, the powdered media typically have longer shelf lives than liquid media, and the media can be sterilized by irradiation (e.g., gamma or ultraviolet irradiation) or ethylene oxide permeation after formulation. However, powdered media have several disadvantages. For example, some components of powdered media become insoluble or aggregate upon lyophilization such that resolubilization is difficult or impossible. Furthermore, powdered media typically comprise fine dust particles which can make them particularly difficult to transfer and/or reconstitute without some loss of material, and which may further make them impractical for use in some settings.

Despite the advancements in rehydratable media, there still exists a need for rapidly dissolving nutritionally complex stable dry powder nutritive media and media supplements, which can be prepared in variable bulk quantities and which are amenable to sterilization.

### SUMMARY

The present disclosure generally relates to nutrient media that are used to facilitate growth of a microorganism (e.g., a bacterium, a yeast, a mold). In particular, the present disclosure provides methods of agglomerating one or more powdered nutrient component to produce a dried, agglomerated nutrient medium. The methods include process conditions that provide agglomerated particles having a selected chemical composition, size, density, water content, or a combination of any two or more of the foregoing properties. In another aspect, the present disclosure further provides a composition comprising a dried agglomerated nutrient medium made according to any method disclosed herein. In another aspect, the present disclosure provides an articles and kits comprising any dried agglomerated nutrient composition disclosed herein.

Advantageously, the compositions of the present disclosure have a chemical composition that facilitates wetting of the surface of the particles by an aqueous solvent. Further advantageously, the compositions of the present disclosure have a density (e.g., a bulk density) selected to facilitate submersion of the particles in an aqueous solvent. Even further advantageously, a dried agglomerated nutrient medium of the present disclosure has a particle size distribution selected to facilitate rapid dissolution of the particles in an aqueous solvent.

In one aspect, the present disclosure provides a method of making a flowable, agglomerated nutrient medium. The method can comprise forming a nutrient medium composition by placing a powdered nutrient into a fluidized bed-type agglomeration chamber, flowing a fluidizing gas through the chamber, and contacting in the agglomeration chamber the powdered nutrient with an aerosol spray of an agglomeration liquid; and collecting the composition from the chamber; wherein the composition comprises a population of agglomerated nutrient medium particles and, optionally, nonagglomerated powdered nutrient; wherein the population comprises at least about 30 weight percent agglomerated nutrient particles having an effective particle diameter of about 250-400 microns. The powdered nutrient comprises a nutrient component that facilitates the growth of a microorganism. In any embodiment of the method, at least about 70 weight percent of particles in the population can have an effective particle diameter of about 105-841 microns. In any of the above embodiments, less than 10 weight percent of particles in the population can have an effective particle diameter of about 105 microns or smaller.

In any of the above embodiments, the method further can comprise isolating a subpopulation of agglomerated nutrient medium particles, the subpopulation having a predetermined effective particle diameter. In some embodiments, isolating a subpopulation can comprise isolating a subpopulation comprises isolating a subpopulation having an effective particle diameter about 149 microns to about 841 microns. In any of the above embodiments, the agglomeration liquid can comprise a solvent with a binder and/or a nutrient that facilitates the growth of a microorganism dissolved therein. In any of the above embodiments, placing a powdered nutrient can comprise placing a predetermined mass of powdered nutrient. In any of the above embodiments, contacting the powdered nutrient with an aerosol spray of an agglomeration liquid can comprise contacting the powdered nutrient with a predetermined volume of agglomeration liquid. In any of the above embodiments, the population of agglomerated nutrient medium particles can have a mean water content of about 5.5 weight percent or less.

In any of the above embodiments, the population of agglomerated nutrient medium particles can have a loose bulk density of about 0.2 to about 0.5 grams per cubic centimeter. In any of the above embodiments, one or more powdered nutrient can comprise a nutrient selected from the group consisting of a protein hydrolysate, a carbohydrate, a salt and a mixture of any two or more of the foregoing powdered nutrients. In any of the above embodiments, the method further can comprise subjecting the dried agglomerated nutrient medium to a process that reduces the number of viable microorganisms in the dried agglomerated nutrient medium. In some embodiments, subjecting the dried agglomerated nutrient medium to a process that reduces the number of viable microorganisms can comprise exposing the dried agglomerated nutrient medium to ionizing radiation or to ethylene oxide vapor. In any of the above embodiments, the powdered nutrient can be selected from the group consisting of a mixture of powders that, when rehydrated, form buffered peptone water medium; a mixture of powders that, when rehydrated, form trypticase soy broth; and a mixture of powders that, when rehydrated, form lactose broth; a mixture of powders that, when rehydrated, form UVM modified Listeria enrichment broth; a mixture of powders that, when rehydrated, form Listeria Enrichment broth; a mixture of powders that, when rehydrated, form Trypticase soy yeast extract broth; a mixture of powders that, when rehydrated, form Rappaport-Vassiliadis enrichment broth; a mixture of powders that, when rehydrated, form Rappaport-Vassiliadis R10 broth; a mixture of powders that, when rehydrated, form Selenite-Cystine broth; a mixture of powders that, when rehydrated, form EC broth; a mixture of powders that, when rehydrated, form Nutrient broth; a mixture of powders that, when rehydrated, form Letheen broth; a mixture of powders that, when rehydrated, form Dey/Engley Neutralizing broth; a mixture of powders that, when rehydrated, form Neutralizing broth; a mixture of powders that, when rehydrated, form Selenite broth; a mixture of powders that, when rehydrated, form Brain Heart Infusion broth; a mixture of powders that, when rehydrated, form half-strength Demi-Fraser broth; a mixture of powders that, when rehydrated, form Frasier broth; and a mixture of powders that, when rehydrated, form Demi-Fraser broth.

In another aspect, the present disclosure provides a composition. The composition can comprise the flowable, dried agglomerated nutrient medium produced by any one of the above methods. In any embodiment of the composition, a specified mass of the dried agglomerated nutrient medium dissolves more rapidly in an aqueous solvent than an equal mass of the powdered nutrient from which the agglomerated nutrient medium was made.

In yet another aspect, the present disclosure provides a composition. The composition can comprise an agglomerated nutrient medium comprising agglomerated particles that, when combined with an aqueous medium, form a mixture that facilitates the growth of a microorganism, wherein at least about 30 weight percent of the particles in the population has an effective particle diameter of about 250-400 microns, wherein the bulk particle density is about 0.2 to about 0.5 grams per cubic centimeter. In any embodiment, at least about 70 weight percent of the particles in the population can have an effective diameter of about 105-841 microns.

In any of the above embodiments of the compositions, the agglomerated nutrient medium can comprise a powdered nutrient, wherein the powdered nutrient is selected from the group consisting of a mixture of powders that, when rehydrated, form buffered peptone water medium; a mixture of powders that, when rehydrated, form trypticase soy broth; and a mixture of powders that, when rehydrated, form lactose broth; a mixture of powders that, when rehydrated, form UVM modified Listeria enrichment broth; a mixture of powders that, when rehydrated, form Buffered Listeria Enrichment broth; a mixture of powders that, when rehydrated, form Trypticase soy yeast extract broth; a mixture of powders that, when rehydrated, form Rappaport-Vassiliadis enrichment broth; a mixture of powders that, when rehydrated, form Rappaport-Vassiliadis R10 broth; a mixture of powders that, when rehydrated, form Selenite-Cystine broth; a mixture of powders that, when rehydrated, form EC broth; a mixture of powders that, when rehydrated, form Nutrient broth; a mixture of powders that, when rehydrated, form Letheen broth; a mixture of powders that, when rehydrated, form Dey/Engley Neutralizing broth; a mixture of powders that, when rehydrated, form Neutralizing broth; a mixture of powders that, when rehydrated, form Selenite broth; a mixture of powders that, when rehydrated, form Brain Heart Infusion broth; a mixture of powders that, when rehydrated, form half-strength Demi-Fraser broth; a mixture of powders that, when rehydrated, form Frasier broth; and a mixture of powders that, when rehydrated, form Demi-Fraser broth.

In yet another aspect, the present disclosure provides a capsule, a tablet, or a sachet comprising any of the above embodiments of the composition.

In yet another aspect, the present disclosure provides a kit. The kit can comprise any of the above embodiments of the capsule, tablet, sachet and/or compositions. In any embodiment of the kit, the composition can be disposed in a package comprising a predetermined mass of the dried agglomerated nutrient medium, wherein the predetermined mass is selected to be mixed with 9.9 mL, 10 mL, 90 mL, 99 mL, 100 mL, 225 mL, 1.0 L, or 3.375 L of aqueous diluent to produce a reconstituted medium capable of facilitating the growth of a microorganism. In any of the above embodiments, the kit further can comprise an article selected from the group consisting of a bag, a bottle, and a sample acquisition device. In any of the above embodiments, the kit further can comprise a selective agent and/or an indicator reagent.

In yet another aspect, the present disclosure provides a thin film culture device. The thin film culture device can comprise a self-supporting waterproof substrate having first and second major surfaces, an adhesive layer disposed on at least a portion of the first major surface, a coating comprising the any of the above embodiments of the composition disposed on a least a portion of the adhesive layer, and a dry, cold-water soluble gelling agent positioned for fluidic contact with the nutrient medium. In any of the above embodiments of the thin film culture device, the device further can comprise a cover sheet coupled to a least a portion of the substrate, the cover sheet having a first side facing the first major surface.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

The terms "comprises" and variations thereof do not have a limiting meaning where these terms appear in the description and claims.

As used herein, "a," "an," "the," "at least one," and "one or more" are used interchangeably. Thus, for example, "a" particle can be interpreted to mean "one or more" particles.

The term "and/or" means one or all of the listed elements or a combination of any two or more of the listed elements.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

Additional details of these and other embodiments are set forth in the accompanying drawings and the description below. Other features, objects and advantages will become apparent from the description and drawings, and from the claims.

### BRIEF DESCRIPTION OF DRAWINGS

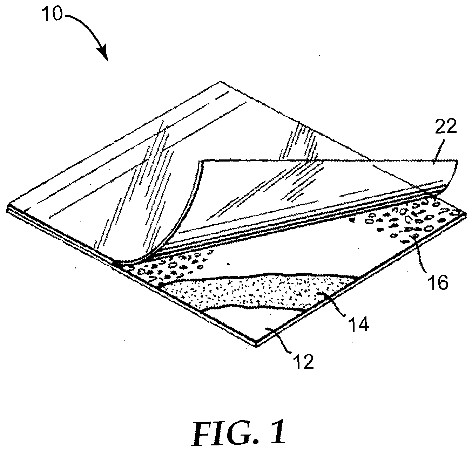

FIG. 1 is a top perspective view, partially in section, of one embodiment of a thin film culture device according to the present disclosure.

### DETAILED DESCRIPTION

Before any embodiments of the present disclosure are explained in detail, it is to be understood that the invention is not limited in its application to the details of construction and the arrangement of components set forth in the following description. The invention is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. It is to be understood that other embodiments may be utilized and structural or logical changes may be made without departing from the scope of the present disclosure.

As used herein, the terms "bulk density", "loose bulk density", and "apparent density" refer interchangeably to untamped bulk density values. Bulk and apparent densities referred to herein can be measured by conventional methods for determining untamped bulk densities such as, for example, the "loose bulk density" method described in the Examples herein.

As used herein, the term "agglomerated particle" refers to a particle formed by the joining or binding together of primary particles whose original identity can still be identified in the final agglomerate form.

All effective particle sizes (i.e., "effective particle diameters") referred to herein are based on the U.S. Standard Sieve Series specifications set by the U.S. Bureau of Standards (see, for example http://www.wirecloth.com/howto/convert/ussieve.html). Thus, for example, a particle having an effective particle diameter of about 400 microns would pass through a 40-mesh screen, which has a specified opening of 400 microns, and would be retained by a 45-mesh screen, which has a specified opening of 354 microns.

In general, the present disclosure relates to flowable, dried agglomerated particles made from water-soluble powders and a method of making said agglomerates. In some embodiments, the method can be used to make a nutrient medium for facilitating the growth of a microorganism. In contrast with at least one of the powders from which the agglomerated particles are made, and in contrast with other agglomerated particle compositions, a population of the inventive agglomerated particles advantageously possess two highly-desirable properties: 1) the agglomerated particles rapidly (i.e., immediately or almost immediately) and substantially quantitatively sink below the surface of an aqueous liquid when poured onto the surface of the aqueous liquid and 2) the agglomerated particles rapidly dissolve into an aqueous liquid. Without being bound by theory, it is believed these highly-desirable properties result from the average size, porosity, surface area, density and/or a unique combination of two or more of the foregoing physical attributes found in populations of agglomerated particles that are produced according to the present disclosure. Thus, in one aspect, the present disclosure provides a nutrient medium that rapidly disperses and dissolves into an aqueous liquid with little or no manual or mechanical agitation.

The present disclosure thus provides a method for the preparation of an agglomerated nutrient medium, the agglomerated medium being suitable to facilitate the recovery and/or growth of a microorganism, the method comprising forming a nutrient medium composition by i) placing a powdered nutrient into a fluidized bed-type agglomeration chamber and ii) contacting in the agglomeration chamber the powdered nutrient with an aerosol spray of an agglomeration liquid. The method further comprises collecting the composition from the chamber. After forming the nutrient medium composition, the composition comprises a population of agglomerated nutrient medium particles and, optionally, nonagglomerated powdered nutrient and the population comprises at least about 30 weight percent agglomerated nutrient particles having an effective particle diameter of about 250-400 microns. According to the method of the present disclosure, the nutrient component (e.g., at least one powdered nutrient) facilitates the growth of a microorganism.

It has been found that application of the agglomeration liquid and wet-massing of the powdered nutrient in a fluidized bed leads to a population of agglomerated particles that is suitable for the preparation of a flowable dried nutrient medium.

Suitable apparatuses for use in the present method include the fluidized bed agglomerators described by Srivastava and Mishra ("Fluid Bed Technology: Overview and Parameters for Process Selection"; Int. J. Pharma. Sci. and Drug Res.; 2010; Vol. 2, pp. 236-246; which is incorporated herein by reference in its entirety. Particularly suitable apparatuses include the "top spray" and "tangential spray" type apparatuses described therein.

Typically, in a top spray agglomeration process, a mass (e.g., a predetermined mass) of a powder component (e.g., one or more powdered nutrient) is loaded into a granulation chamber. The powder component is fluidized by passing a fluidization gas (e.g. air, nitrogen) through the powder component to create a fluidized bed of the powder component. While the powder is fluidized, an agglomeration liquid is sprayed into the agglomeration chamber where it contacts the fluidized powder component and/or agglomerated nutrient medium particles. Upon contact, the agglomeration liquid can form a coating on at least a portion of the surface of a particle of the powder component which may contact another powder particle to form an agglomerated particle. Subsequently and/or simultaneously, the fluidizing gas facilitates the evaporation of the agglomeration liquid, thereby drying the agglomerated particle. Thus, without being bound by theory, agglomeration takes place via one or more of the following mechanisms: 1) Immobile agglomeration liquid on the surface of a particle forms bridges (e.g., due to adhesional and cohesional forces) between two or more particles, 2) Mobile agglomeration liquid (e.g., moving by interfacial and/or capillary force between adjacent particles) forms bridges between two or more particles and 3) Solid bridges are formed between two or more particles, due to the crystallization of dissolved substances (e.g., dissolved into the agglomeration liquid as the agglomeration liquid contacts the surface of a particle) during drying.

A person having ordinary skill in the art will recognize, depending on the size of the apparatus employed, the mass of the powder composition loaded the agglomeration chamber as well as the flow rate of the fluidizing gas and the flow rate of the agglomeration liquid may vary.

A method according to the present disclosure is used to make an agglomerated nutrient medium (e.g., a dried, wet-agglomerated nutrient medium). The agglomerated nutrient medium may be an agglomerated microbiological nutrient medium that facilitates the growth of a microorganism (e.g., a bacterium, a yeast, a mold). A method of the present disclosure comprises placing a powdered nutrient into a fluidized bed-type agglomeration chamber. The powdered nutrient comprises a nutrient component that supports the growth of a microorganism. In any embodiment, placing a powdered nutrient into a fluidized bed-type agglomeration chamber can comprise placing a predetermined mass of powdered nutrient into the agglomeration chamber. The nutrient component can be any suitable nutrient component described herein. In any embodiment, the nutrient component can comprise a substantially uniform mixture of two or more powdered nutrients.

In any embodiment, a method of the present disclosure comprises contacting in the agglomeration chamber the powdered nutrient with an aerosol spray of an agglomeration liquid. In any embodiment, contacting the powdered nutrient with the agglomeration liquid can comprise contacting the powdered nutrient (e.g., a predetermined mass of powdered nutrient) with a predetermined volume of agglomeration liquid. A person having ordinary skill in the art will recognize that certain process parameters (e.g., amount of powder particles, fluidization of the particles, atomization pressure, temperature of the agglomeration chamber, ratio of agglomeration liquid volume to the powdered nutrient mass, and the flow rate of the agglomeration liquid) can be adjusted to promote increased contact between particles that have agglomeration liquid disposed thereon and, thereby facilitate the formation of larger agglomerates Conversely, a person having ordinary skill in the art will recognize that certain process parameters, including those listed above, can be adjusted to decrease contact between particles that have agglomeration liquid disposed thereon and, thereby facilitate the formation of smaller agglomerates. In particular, the atomization pressure of the agglomeration liquid can be increased in order to increase the percentage of smaller agglomerates (e.g., about 105 to about 400 microns effective diameter) in the composition formed by the agglomeration process.

The fluidizing gas that is fed to the fluidized bed is usually air, although any other gas inert to the powders being agglomerated can be used, for example nitrogen. The fluidizing gas generally is introduced into the agglomeration chamber (e.g., through one or more inlets) at an inlet temperature of about 0° C to about 140° C., preferably about 20° C to about 140° C. It may be preferred that the fluidizing gas introduced into the agglomeration chamber is heated, for example to about 50° C to about 140° C, preferably about 50° C to about 90° C.

In any embodiment, the powder ingredients used to make agglomerated particles according to the present disclosure can be blended in the agglomeration chamber (e.g., at room temperature) for a predefined period of time (e.g., 20 minutes) before the agglomeration liquid is introduced into the chamber. Advantageously, this blending step can result in a more-uniform mixture of the respective powder ingredients which, when agglomerated, can result in substantial uniformity in the agglomerated particles, as evidenced by Example 11 described herein.

Typically, the fluidizing gas passes through and out of the agglomeration chamber during the agglomeration process. Thus, the agglomeration chamber typically has one or more exit port through which the fluidizing gas passes out of the agglomeration chamber. As the fluidizing gas passes through the agglomeration chamber, it causes the evaporation of the agglomeration liquid and, thereby, its temperature drops. Thus, in any embodiment, the temperature of the fluidizing gas as it exits the agglomeration chamber can be lower than the temperature of the fluidizing gas at the inlet port. In any embodiment, the temperature of the fluidizing gas as it exits the agglomeration chamber can be about 35° C to about 50° C.

In any embodiment, the agglomeration process can be controlled by adjusting the inlet temperature of the fluidizing gas and maintaining the spray rate of the agglomeration liquid to maintain a second temperature of the fluidizing gas (e.g. within the spraying zone of the agglomeration chamber) within a predetermined temperature range (e.g., about 35° C to about 50° C.

The volume and flow rate of the agglomeration liquid introduced into the agglomeration chamber are also important parameters in order for proper application of the agglomeration liquid to wet-mass the powdered nutrient. Normally, the agglomeration liquid is sprayed on the composition through one or more aerosol nozzles. A person skilled in the art will recognize the liquid flow rate can be adjusted, for example, by controlling the liquid pressure at the nozzle. In any embodiment, the liquid flow rate can be controlled to maintain a predetermined outlet temperature of the fluidizing gas. In any embodiment, contacting in an agglomeration chamber the powdered nutrient (e.g., a predetermined mass of powdered nutrient) with an aerosol spray of agglomeration liquid can comprise contacting the powdered nutrient with a predetermined volume of agglomeration liquid.

Optionally, after removal from the agglomeration chamber, the agglomerated nutrient medium may be subjected to drying in a suitable drying chamber. The drying chamber may be operated batch wise or continuously and, optionally, may be segregated into two or more independently-controlled drying zones, the zones being operated at different temperatures. In any embodiment, the drying conditions (e.g., temperature, time, and gas or air flow) can be selected to produce agglomerated nutrient medium particles having a preselected average water content and/or a preselected range of water content.

In any of the above embodiments, a method of the present disclosure further can comprise subjecting the dried agglomerated nutrient medium to a process that reduces the number of viable microorganisms in the dried agglomerated nutrient medium. In any embodiment, the dried agglomerated nutrient medium can be treated with ethylene oxide vapor in order to reduce the number of viable microorganisms according to methods that are known in the art. After treatment with ethylene oxide vapor, the nutrient medium can be allowed to aerate before use, in order to reduce or eliminate residual ethylene oxide in the ethylene oxide-treated agglomerated medium. In any embodiment, the dried agglomerated nutrient medium can be treated with ionizing radiation (e.g., gamma radiation) or a source of ultraviolet light in order to reduce the number of viable microorganisms according to methods that are known in the art. The dried, agglomerated medium can be exposed to a source of gamma irradiation as described in U.S. Patent No. 6,383,810, which is incorporated herein by reference in its entirety.

### Nutrient component

Methods of the present disclosure comprise a step of contacting in the agglomeration chamber the powdered nutrient with an aerosol spray of an agglomeration liquid. The powdered nutrient comprises at least one nutrient component that facilitates the growth of a microorganism (e.g., a bacterium, a yeast, a mold). In any embodiment, the nutrient component may comprise a mixture of two or more nutrients that facilitate the growth of a microorganism. Non-limiting examples of suitable nutrients include a carbohydrate (e.g., a monosaccharide, a disaccharide, a trisaccharide, an oligosaccharide, or a polysaccharide), a protein, a protein hydrolysate, a cellular extract (e.g., yeast extract), a salt, a buffer component, a selective agent (e.g., an antibiotic), and a combination of any two or more of the foregoing nutrients.

In any embodiment, the nutrient component can be a substantially dry formulation (e.g., a substantially dry powder). Powdered media are typically produced by admixing the dried components of the culture medium via a mixing process, e.g., ball-milling, or by lyophilizing premade liquid culture medium. An exemplary nutrient medium that is used in the method of the present disclosure is buffered peptone water, which is commercially-available as a powder from a variety of sources including, for example, 3M Health Care (St. Paul, MN), Oxoid Limited (Hampshire, UK) and Sigma-Aldrich (St. Louis, MO).

Other powdered nutrient media formulations that can be agglomerated according to the present disclosure include trypticase soy broth, lactose broth, UVM modified Listeria enrichment broth, Buffered Listeria Enrichment broth, Trypticase soy yeast extract broth, Rappaport-Vassiliadis enrichment broth, a mixture of powders that, when rehydrated, form Rappaport-Vassiliadis R10 broth; a mixture of powders that, when rehydrated, form Selenite-Cystine broth; a mixture of powders that, when rehydrated, form EC broth; a mixture of powders that, when rehydrated, form Nutrient broth; a mixture of powders that, when rehydrated, form Letheen broth; a mixture of powders that, when rehydrated, form Dey/Engley Neutralizing broth; a mixture of powders that, when rehydrated, form Neutralizing broth; Selenite broth, Brain Heart Infusion broth, half-strength Demi-Fraser broth, Frasier broth, Demi-Fraser broth, and other microbiological nutrient medium formulations.

The powder ingredients fed to the fluidized bed typically have a mean particle diameter in the range 5 to 200 µm, although powder ingredients with even larger particle sizes (e.g., up to 300 µm or even 400 µm) can be used. The powder ingredients fed to the fluidized bed generally form at least 60% by weight of the agglomerated nutrient medium and often over 80%, up to 95 or 100% by weight of the agglomerated nutrient medium on a dry weight basis.

### Agglomeration liquid

An agglomeration liquid is sprayed into the agglomeration chamber to promote agglomeration of the smaller individual particles (e.g., powder particles) of the nutrient component into larger agglomerated particles. In any embodiment, the agglomeration liquid can comprise water (e.g., distilled and/or deionized water). In any embodiment, the agglomeration liquid can consist essentially of water (e.g., distilled and/or deionized water). In any embodiment, the agglomeration liquid can consist of water (e.g., distilled and/or deionized water). In any embodiment, the agglomeration liquid may comprise an organic solvent (e.g., ethanol, isopropanol).

In any embodiment, the agglomeration liquid may comprise a dissolved material (e.g., a nutrient, nutrient medium, and/or a binder, as discussed below). In any embodiment, the agglomeration liquid can for example be at a temperature of from 0° or 20°C, up to 100° or 110°C when it is injected into the agglomeration chamber. When the agglomeration liquid contains no dissolved material (e.g., to aid agglomeration) it may be preferred that the agglomeration liquid (e.g., water) is heated, for example to 50-110° C. Steam is generally at least as effective as hot water in promoting agglomeration.

Optionally, in any embodiment, a binder, especially a water-soluble binder, may be useful to facilitate the agglomeration of the nutrient component. The binder may be added (e.g., in the form of substantially dry particles such as a powder, for example) to the nutrient component or it may be dissolved or suspended in the agglomeration liquid.

Optionally, in any embodiment, the agglomeration liquid further may comprise a wetting agent (e.g., a surfactant such as TWEEN) to facilitate the dissolution (e.g., at least partial dissolution) of the agglomerated particles.

Nonlimiting examples of suitable binders include biocompatible polymers (e.g. proteins, polyethylene glycol, polyvinylpyrollidone, polyvinyl alcohol, polysaccharides, dextrans, dextrins, maltodextrins, microcrystalline cellulose, hydroxypropylcellulose, hydroxypropyl cellulose, hydroxypropylmethylcellulose, methylcellulose, starch, and biocompatible derivatives of any of the foregoing polymers) and sugars (e.g., dextrose, fructose, glucose, inositol, erythritol, isomalt, lactitol, lactose, maltitol, maltose, mannitol, sorbitol, sucrose, tagatose, trehalose, and xylitol). In any embodiment, the binder may be a substance that facilitates the growth of a microorganism (e.g., a complete medium or diluent containing at least one nutrient). By way of example, a suitable agglomeration liquid for agglomerated buffered peptone water medium can be an solution of 1-50 weight percent buffered peptone water and a suitable agglomeration liquid for agglomerated trypticase soy broth medium can be an aqueous solution of 1-50 weight percent trypticase soy medium.

The concentration of the binder in the composition comprising the nutrient may vary over a great range depending on the particular binder employed, but in general it is between from about 0.1% to about 40% w/w such as, e.g. from about 0.2 to about 35% w/w, from about 0.3 to about 30% w/w or from about 0.4 to about 25% w/w or from about 0.4 to about 24.2% w/w. In the case of sorbitol, the concentration is normally about 20-30% w/w and in the case of sugar alcohols other than sorbitol; the concentration is normally in the higher range such as from about 30 to about 40% w/w.

Preferably, the agglomeration liquid is an aqueous medium. In the case where the binder is included in the agglomeration liquid, the agglomeration liquid is prepared by dissolving the binder in water. Alternatively the binder can be admixed in a dry form to the powder.

In any embodiment, the agglomeration liquid may comprise a nutrient medium that facilitates the growth of a microorganism. For example, in one embodiment, the agglomeration liquid may comprise buffered peptone water broth (water, 10.0 g/L peptone, 5.0 g/L sodium chloride, 9.0 g/L disodium hydrogen orthophosphate 12H₂O, and 1.5 g/L potassium dihydrogen orthophosphate).

In any embodiment of the method, the agglomeration liquid may comprise at least one dissolved nutrient that facilitates the growth of a microorganism. The dissolved nutrient may comprise a protein (e.g., a mixture of protein, a purified protein, and/or a protein hydrolysate). In one embodiment, the agglomeration liquid comprises buffered peptone water.

A person having ordinary skill in the art will recognize that at least some of the process conditions (e.g., mass of nutrient powder used in the process, mass of agglomeration liquid used in the process, agglomeration liquid spray rate, and/or the temperature in the agglomeration chamber) can affect one or more of the physicochemical properties of the resulting agglomerated nutrient medium particles produced by the method if the present disclosure. These physicochemical properties include, but are not limited to the average effective particle diameter, the particle shape, the average particle mass, the average particle moisture content, the range of effective particle diameters, the range of particle masses, and/or the range of particle moisture content. One or a combination of any two or more of the physicochemical properties can affect the hydrodynamic properties (e.g., particle sink rate in an aqueous liquid, particle dissolution time in an aqueous liquid) of the dried, agglomerated nutrient medium particles.

In any embodiment, a method according to the present disclosure yields a population of dried, agglomerated nutrient medium particles having an average effective particle diameter and a first particle size distribution range. In any embodiment, the first particle size distribution range can comprise agglomerated particles having an effective particle diameter from about 105 microns to about 2000 microns. In any embodiment, the first particle size distribution range can comprise agglomerated particles having an effective particle diameter from about 105 microns to about 1000 microns.

In any embodiment, a composition of agglomerated particles produced using a method of the present disclosure may comprise a population of agglomerated particles wherein at least about 70 weight percent of the agglomerated particles has an effective particle diameter of about 105 microns to about 841 microns. In any embodiment, the agglomerated particles produced using a method of the present disclosure may comprise a first population of agglomerated particles wherein less than 10 weight percent of the agglomerated particles has an effective particle diameter of about 105 microns or smaller.

In any embodiment, the process conditions for a method according to the present disclosure can be selected to limit the weight percent of relatively large (e.g., having an effective particle diameter of >841 microns) particles in the composition produced according to the method of the present disclosure. For example, the process can yield a population of agglomerated particles with less than 4 weight percent of particles having an effective particle diameter > 841 microns.

In any embodiment, the process conditions for a method according to the present disclosure can be selected to enrich the proportion of particles having an effective particle diameter of 400-841 microns, inclusive. For example, the process conditions can be selected to yield a population of agglomerated particles with about 1.5 weight percent to about 40 weight percent of the agglomerated particles having an effective particle diameter of 400-841 microns, inclusive.

In any embodiment, the process conditions for a method according to the present disclosure can be selected to enrich the proportion of particles having an effective particle diameter of 250-400 microns, inclusive. For example, the process conditions can be selected to yield a population of agglomerated particles with about 35 weight percent to about 55 weight percent of the agglomerated particles having an effective particle diameter of 250-400 microns, inclusive.

In any embodiment, the process conditions for a method according to the present disclosure can be selected to enrich the proportion of particles having an effective particle diameter of 105-250 microns, inclusive. For example, the process conditions can be selected to yield a population of agglomerated particles with about 15 weight percent to about 55 weight percent of the agglomerated particles having an effective particle diameter of 105-250 microns, inclusive.

In any embodiment, the process conditions for a method according to the present disclosure can be selected to limit the weight percent of relatively small (e.g., having an effective particle diameter of <105 microns) particles in the composition produced according to the method of the present disclosure. For example, the process can yield a population of agglomerated particles with less than 5 weight percent of particles having an effective particle diameter <105 microns. In some embodiments, the process can yield a population of agglomerated particles with less than 1 weight percent of particles having an effective particle diameter <105 microns.

Thus, in a preferred embodiment, the process conditions for a method according to the present disclosure can be selected to yield a population of agglomerated particles having about 0-4 weight percent of the particles having an effective particle diameter of >841 microns; about 1.5-40 weight percent of the particles having an effective particle diameter of 400-841 microns, inclusive; about 35-50 weight percent of the particles having an effective particle diameter of 250-400 microns, inclusive; about 15-55 weight percent of the particles having an effective particle diameter of 105-250 microns, inclusive; and <5 weight percent of the particles having an effective particle diameter of <105 microns.

In any embodiment, the method further can comprise isolating a subpopulation of the dried, agglomerated nutrient medium particles, the subpopulation having a preselected average effective particle diameter and/or having an effective particle diameter that falls within a second particle size distribution range. The subpopulation can be isolated using particle size-selection methods known in the art (e.g., using sieves that permit the retention or passage of particles with predetermined effective particle diameters. In any embodiment, the subpopulation may include agglomerated particles having an effective particle diameter from about 105 microns to about 841 micron, inclusive. In any embodiment, the subpopulation may include agglomerated particles having an effective particle diameter from about 105 microns to about 400 microns, inclusive. In any embodiment, the subpopulation may include agglomerated particles having an effective particle diameter from about 125 microns to about 400 microns, inclusive. In any embodiment, the subpopulation may include agglomerated particles having an effective particle diameter from about 150 microns to about 250 microns, inclusive.

In another aspect, the present disclosure also provides a composition. The composition comprises a flowable, agglomerated nutrient medium. The composition can be prepared using any embodiment of the method disclosed herein. The composition can be used to prepare nutrient medium to facilitate the recovery and/or growth of a microorganism in a sample (e.g., a clinical sample, an environmental sample, a food sample, a beverage sample, a water sample). Moreover, the composition can be used in an article for culturing microorganisms.

Advantageously, a composition of the present disclosure is flowable (e.g., can be poured from one container into another), is substantially free of dust (e.g., the smaller particles of nutrient from which the agglomerated medium is made and, when the agglomerated medium is made from a substantially dry starting material that includes at least one water-soluble powder, the agglomerated medium can dissolve in water substantially faster than the starting material.

In any embodiment of the method, the dried agglomerated nutrient medium particles of the composition can have selected mean water content (e.g., expressed in weight percent water) that facilitates the dispersion and dissolution of the composition in an aqueous solvent. The mean water content can be measured, for example, using the Karl Fischer titration method. In any embodiment, the mean weight percent water can be preselected to be less than about 5 weight percent water. In any embodiment, the mean weight percent water can be preselected to be less than or equal to about 3 weight percent water. A person having ordinary skill in the art will recognize the mean water content can be adjusted, for example, by controlling the amount of water used to wet-mass the agglomerated particles and/or by controlling drying conditions during and/or after the agglomeration process.

In any embodiment of the method, the dried agglomerated nutrient medium particles can have a loose bulk density that facilitates dispersion of the agglomerated particles in an aqueous solvent. The loose bulk density of the dried, agglomerated nutrient medium particles can be measured, for example, by pouring a predefined volume (e.g., 20 mL) of the particles into a 50-mL graduated cylinder, and measuring the mass of the gravity-packed particles in order to calculate the average density. In any embodiment, the loose bulk density can be from about 0.2 grams per cubic centimeter to about 0.5 grams per cubic centimeter.

Advantageously, compositions of the present disclosure disperse and dissolve rapidly in an aqueous solvent. When the dissolution time is measured by pouring 2.55g to 4.44 grams of a dried, agglomerated nutrient medium (e.g., a dried, agglomerated nutrient medium used to make buffered peptone water, trypticase soy broth, or modified Listeria recovery medium, for example) into a vessel containing 100 mL of deionized water and holding the vessel at room temperature without stirring, the dried agglomerated medium can dissolve in less than 5 minutes. In some embodiments, the dried agglomerated medium can dissolve in less than 4 minutes. In some embodiments, the dried agglomerated medium can dissolve in less than 3 minutes. In some embodiments, the dried agglomerated medium can dissolve in less than 2 minutes. In some embodiments, the dried agglomerated medium can dissolve in less than 1 minute.

The present disclosure shows that the dissolution time for a composition of agglomerated particles of nutrient medium can be selected by adjusting the process conditions and/or using an optional isolation process (e.g., sieving) to obtain a composition having a particular effective particle diameter. For example, an agglomerated Buffered Peptone Water medium, having an effective particle diameter between 400 and 841 microns, inclusive, can dissolve in about 90-120 seconds in room temperature water, as shown in Example 8; an agglomerated Buffered Peptone Water medium, having an effective particle diameter between 250 and 400 microns, inclusive, can dissolve in about 30 seconds in room temperature water, as shown in Example 8; agglomerated Buffered Peptone Water medium, having an effective particle diameter between 105 and 250 microns, inclusive, can dissolve in about 15 seconds in room temperature water, as shown in Example 8; and agglomerated Buffered Peptone Water medium, having an effective particle diameter <105 microns can dissolve in about 90-120 seconds in room temperature water, as shown in Example 8; when the mixture of powders used to make the agglomerated BPW nutrient medium requires >10 minutes to dissolve in room temperature water.

In a preferred embodiment, the composition of the present disclosure is made from powders that can be reconstituted to form a recovery medium (i.e., an aqueous medium that can be used to permit microorganisms to recover from chemical and/or environmental stress). In some embodiments, the recovery medium can be used to culture (e.g., grow) microorganism. Alternatively, or additionally, the recovery medium can be used as a diluent. A recovery medium that is particularly preferred to facilitate the recovery and/or growth of a stressed microorganism is Buffered Peptone Water (BPW) medium, which comprises a protein hydrolysate, salt, and a phosphate buffer system adjusted to pH 7.2. A specified mass of the agglomerated BPW medium can dissolve in as little as 15 seconds, as shown in the Examples herein.

In any embodiment, a composition of agglomerated nutrient medium according to the present disclosure can be used to make a capsule, a tablet, or a sachet according to methods that are known in the art. Advantageously, the capsule, tablet, or sachet can provide a convenient means to deliver predetermined mass (e.g., "unit dose") of the composition to a container in which the composition will be hydrated to form the reconstituted nutrient medium.

In yet another aspect, the present disclosure provides an article for culturing a microorganism. A nonlimiting example of an article according to the present disclosure is a thin film culture device similar to the powder-coated devices described in U.S. Patent Nos. 4,565,783 and 5,089,413; which are incorporated herein by reference in their entirety. The dried, agglomerated nutrient medium of the present disclosure can be used in place of the powdered nutrients in the thin film culture devices.

FIG. 1 shows one embodiment of a thin film culture device according to the present disclosure. The culture device 10 includes a body member comprising a self-supporting water-proof substrate 12 having upper and lower surfaces. Substrate 12 is preferably a relatively stiff film of a material such as polyester, polypropylene or polystyrene which will not absorb or otherwise be affected by water. Polyester films approximately 0.004 to 0.007 inch (0.1 to 0.18 mm) thick, polypropylene films approximately 0.004 to 0.008 inch (0.1 to 0.2 mm) thick and polystyrene films approximately 0.015 inch (0.38 mm) thick have been found to work well. Other suitable substrates include paper with a polyethylene or other water-proof coating. An example of a suitable polyethylene-coated paper substrate is "Schoeller Type MIL" photoprint paper (commercially available from Schoeller Pulaski, New York, NY). The substrate 12 may be either transparent or opaque, depending on whether one wishes to view bacterial colonies through the substrate. To facilitate the counting of bacterial colonies, the substrate 12 preferably has a square grid pattern printed thereon as described in U.S. Patent No. 4,565,783, for example.

Substrate 12 is coated on its upper surface with a layer of an adhesive 14 which serves to hold the dry gelling agent and/or nutrients in a uniform monolayer for easy hydration. Adhesive 14 should be water-insoluble and non-inhibitory to the growth of microorganisms. Preferably, the adhesive is sufficiently transparent when wet to enable the viewing of bacterial colonies through the film coated with the adhesive. It is preferred that adhesive 14 be pressure-sensitive. However, heat-activated adhesives wherein a lower melting substance is coated onto a higher melting substance may also be used. Water-activated adhesives such as mucilage may also be useful.

Adhesive 14 should be coated onto substrate 12 in a thickness which is preferably less than the diameter of the particles of the powdered gelling agent and/or nutrients. The object is to apply enough adhesive to adhere the particles to the substrate but not so much that the particles become completely embedded in the adhesive. A uniform monolayer of powder 16 is desired with sufficient surface area exposed for hydration. Generally, an adhesive layer in the thickness range of 0.0002 to 0.0005 inch is suitable. The powders, including the dried, agglomerated nutrient medium of the present disclosure, can be coated onto the adhesive layer as described in U.S. Patent No. 4,565,783, for example.

Adhered to one edge of spacer 18 of the body member is an optional cover sheet 22. Cover sheet 22 is preferably transparent to facilitate counting of the bacterial colonies and is substantially impermeable to bacteria and water vapor. As used in the specification and claims, "substantially impermeable to bacteria and moisture vapor" designates cover sheets which prevent undesired contamination of the dehydrated medium during storage and use of the devices and which will provide an environment which will support the growth of microorganisms during the incubation period. Generally, it will have the same properties as substrate 12, but need not be as stiff.

Although the illustrated embodiment of FIG. 1 includes a cover sheet 22 attached to the device, it is also contemplated within the scope of the invention that the powder-containing embodiments may be uncovered and simply placed in a sterile environment during storage and incubation. Optionally, the cover sheet 22 further may comprise a layer of adhesive (not shown) and powder (not shown). The adhesive and powder adhered to the cover sheet can have similar (or identical) properties and/or composition as the adhesive 14 and powder 16 described above and can be applied to the cover sheet in the same manner as adhesive 14 and powder 16 are applied to the substrate 12.

In another aspect, the present disclosure provides a kit. The kit can comprise any composition comprising the dried, agglomerated nutrient medium described herein. In any embodiment, the kit may include instructions for using the dried, agglomerated nutrient medium. In any embodiment, the kit may further comprise an article for obtaining a sample, processing a sample, and/or for culturing a microorganism. The article can be selected from the group consisting of a bag, a bottle, a sample acquisition device (e.g., a pipette, a swab, a sponge), and a combination of any two or more of the foregoing articles.

In any of the above embodiments, the kit further may comprise a selective agent (e.g., an antibiotic) that favors the growth of one type of microorganism over another type of microorganism. In any embodiment, the selective agent can be provided in a solution or in a substantially dry form. In any embodiment, the selective agent can be provided in a unit-dose form (e.g., a tablet, tube, or ampoule) that can be added to a predetermined volume of sample.

In any embodiment, the kit further may comprise an indicator reagent used to detect a presence or an absence of a microorganism in a sample. Suitable indicator reagents include, for example, a pH indicator (e.g., phenol red, chlorophenol red, methyl red, neutral red, bromthymol blue, bromcresol purple), a redox indicator (e.g., triphenyltetrazolium chloride, methylene blue), a chromogenic enzyme substrate (e.g., a chromogenic enzyme substrate to detect a glycosidase enzyme activity, a phosphatase enzyme activity, a lipase enzyme activity, or an aminopeptidase enzyme activity), or a fluorogenic enzyme substrate (e.g., a fluorogenic enzyme substrate to detect a glycosidase enzyme activity, a phosphatase enzyme activity, a lipase enzyme activity, or an aminopeptidase enzyme activity).

In any of the above embodiments, the kit may comprise a package comprising a predetermined mass of the dried agglomerated nutrient medium. In any embodiment, the predetermined mass can be an amount sufficient to be mixed with a predetermined volume (e.g., 9.9 mL, 10 mL, 90 mL, 99 mL, 100 mL, 225 mL, 1.0 L, or 3.375 L) of aqueous diluent to produce a reconstituted medium capable of facilitating the growth of a microorganism. In any embodiment, the predetermined mass of the composition can be provided in the kit in the form of a capsule, a tablet, or a sachet.

Certain embodiments of the methods, compositions, articles, and kits of the present disclosure are set forth in the following list of embodiments.

### EMBODIMENTS

Embodiment A is a method of making a flowable, agglomerated nutrient medium, the method comprising:
   forming a nutrient medium composition by:
      placing a powdered nutrient into a fluidized bed-type agglomeration chamber; wherein the powdered nutrient comprises a nutrient component that facilitates the growth of a microorganism;
      flowing a fluidizing gas through the chamber,
      contacting in the agglomeration chamber the powdered nutrient with an aerosol spray of an agglomeration liquid; and
   collecting the composition from the chamber;
   wherein the composition comprises a population of agglomerated nutrient medium particles and, optionally, nonagglomerated powdered nutrient;
   wherein the population comprises at least about 30 weight percent agglomerated nutrient particles having an effective particle diameter of about 250-400 microns.
Embodiment B is the method of Embodiment A, wherein at least about 70 weight percent of particles in the population has an effective particle diameter of about 105-841 microns.
Embodiment C is the method of Embodiment A or Embodiment B, wherein less than 10 weight percent of particles in the population has an effective particle diameter of about 105 microns or smaller.
Embodiment D is the method of any one of the preceding Embodiments, further comprising isolating a subpopulation of agglomerated nutrient medium particles, the subpopulation having a predetermined effective particle diameter.
Embodiment E is the method of Embodiment D, wherein isolating a subpopulation comprises isolating a subpopulation having an effective particle diameter about 149 microns to about 841 microns.
Embodiment F is the method of any one of the preceding Embodiments, wherein the agglomeration liquid consists of water.
Embodiment G is the method of any one of Embodiments A through E, wherein the agglomeration liquid comprises a solvent with a binder and/or a nutrient that facilitates the growth of a microorganism dissolved therein.
Embodiment H is the method of Embodiment G, wherein the powdered nutrient comprises a substantially uniform mixture of two or more powdered nutrients.
Embodiment I is the method of any one of the preceding Embodiments, wherein placing a powdered nutrient comprises placing a predetermined mass of powdered nutrient.
Embodiment J is the method of any one of the preceding Embodiments, wherein contacting the powdered nutrient with an aerosol spray of an agglomeration liquid comprises contacting the powdered nutrient with a predetermined volume of agglomeration liquid.
Embodiment K is the method of any one of the preceding Embodiments, wherein the fluidizing gas has a first temperature as it enters the agglomeration chamber, wherein the first temperature of the fluidizing gas is about 50° C to about 90° C.
Embodiment L is the method of Embodiment J, wherein the fluidizing gas has a second temperature in a spraying zone of the agglomeration chamber, wherein the second temperature of the fluidizing gas is about 35° C to about 50° C.
Embodiment M the method of any one of the preceding Embodiments, wherein the population of agglomerated nutrient medium particles has a mean water content of about 5.5 weight percent or less.
Embodiment N is the method of any one of the preceding Embodiments, wherein the population of agglomerated nutrient medium particles has a loose bulk density of about 0.2 to about 0.5 grams per cubic centimeter.
Embodiment O is the method of any one of the preceding Embodiments, wherein one or more powdered nutrient comprises a nutrient selected from the group consisting of a protein hydrolysate, a carbohydrate, a salt and a mixture of any two or more of the foregoing powdered nutrients.
Embodiment P is the method of any one of the preceding Embodiments, further comprising the step of subjecting the dried agglomerated nutrient medium to a process that reduces the number of viable microorganisms in the dried agglomerated nutrient medium.
Embodiment Q is the method of Embodiment P, wherein subjecting the dried agglomerated nutrient medium to a process that reduces the number of viable microorganisms comprises exposing the dried agglomerated nutrient medium to ionizing radiation or to ethylene oxide vapor.
Embodiment R is the method of any one of the preceding Embodiments, wherein the powdered nutrient is selected from the group consisting of a mixture of powders that, when rehydrated, form buffered peptone water medium; a mixture of powders that, when rehydrated, form trypticase soy broth; and a mixture of powders that, when rehydrated, form lactose broth; a mixture of powders that, when rehydrated, form UVM modified Listeria enrichment broth; a mixture of powders that, when rehydrated, form Buffered Listeria Enrichment broth; a mixture of powders that, when rehydrated, form Trypticase soy yeast extract broth; a mixture of powders that, when rehydrated, form Rappaport-Vassiliadis enrichment broth; a mixture of powders that, when rehydrated, form Rappaport-Vassiliadis R10 broth; a mixture of powders that, when rehydrated, form Selenite-Cystine broth; a mixture of powders that, when rehydrated, form EC broth; a mixture of powders that, when rehydrated, form Nutrient broth; a mixture of powders that, when rehydrated, form Letheen broth; a mixture of powders that, when rehydrated, form Dey/Engley Neutralizing broth; a mixture of powders that, when rehydrated, form Neutralizing broth; a mixture of powders that, when rehydrated, form Selenite broth; a mixture of powders that, when rehydrated, form Brain Heart Infusion broth; a mixture of powders that, when rehydrated, form half-strength Demi-Fraser broth; a mixture of powders that, when rehydrated, form Frasier broth; and a mixture of powders that, when rehydrated, form Demi-Fraser broth.
Embodiment S is a composition comprising the flowable, dried agglomerated nutrient medium produced by the method of any one of the preceding Embodiments.
Embodiment T is the composition of Embodiment S, wherein a specified mass of the dried agglomerated nutrient medium dissolves more rapidly in an aqueous solvent than an equal mass of the powdered nutrient from which the agglomerated nutrient medium was made.
Embodiment U is a composition comprising an agglomerated nutrient medium comprising agglomerated particles that, when combined with an aqueous medium, form a mixture that facilitates the growth of a microorganism, wherein at least about 30 weight percent of the particles in the population has an effective particle diameter of about 250-400 microns, wherein the bulk particle density is about 0.2 to about 0.5 grams per cubic centimeter.
Embodiment V is the composition of Embodiment U, wherein at least about 70 weight percent of the particles in the population has an effective diameter of about 105-841 microns.
Embodiment W is the composition of Embodiment U or Embodiment V, wherein the agglomerated nutrient medium comprises a powdered nutrient, wherein the powdered nutrient is selected from the group consisting of a mixture of powders that, when rehydrated, form buffered peptone water medium; a mixture of powders that, when rehydrated, form trypticase soy broth; and a mixture of powders that, when rehydrated, form lactose broth; a mixture of powders that, when rehydrated, form UVM modified Listeria enrichment broth; a mixture of powders that, when rehydrated, form Buffered Listeria Enrichment broth; a mixture of powders that, when rehydrated, form Trypticase soy yeast extract broth; a mixture of powders that, when rehydrated, form Rappaport-Vassiliadis enrichment broth; a mixture of powders that, when rehydrated, form Rappaport-Vassiliadis R10 broth; a mixture of powders that, when rehydrated, form Selenite-Cystine broth; a mixture of powders that, when rehydrated, form EC broth; a mixture of powders that, when rehydrated, form Nutrient broth; a mixture of powders that, when rehydrated, form Letheen broth; a mixture of powders that, when rehydrated, form Dey/Engley Neutralizing broth; a mixture of powders that, when rehydrated, form Neutralizing broth; a mixture of powders that, when rehydrated, form Selenite broth; a mixture of powders that, when rehydrated, form Brain Heart Infusion broth; a mixture of powders that, when rehydrated, form half-strength Demi-Fraser broth; a mixture of powders that, when rehydrated, form Frasier broth; and a mixture of powders that, when rehydrated, form Demi-Fraser broth.
Embodiment X is a capsule, a tablet, or a sachet comprising the composition of any one of Embodiments S through W.
Embodiment Y is a kit comprising the capsule, tablet, sachet, and/or composition of any one of Embodiments S through X.
Embodiment Z is the kit of Embodiment Y, wherein the composition is disposed in a package comprising a predetermined mass of the dried agglomerated nutrient medium, wherein the predetermined mass is selected to be mixed with 9.9 mL, 10 mL, 90 mL, 99 mL, 100 mL, 225 mL, 1.0 L, or 3.375 L of aqueous diluent to produce a reconstituted medium capable of facilitating the growth of a microorganism.
Embodiment AA is the kit of Embodiment Y or Embodiment Z, further comprising an article selected from the group consisting of a bag, a bottle, and a sample acquisition device.
Embodiment BB is the kit of any one of Embodiments Y through AA, further comprising a selective agent and/or an indicator reagent.
Embodiment CC is a thin film culture device, comprising:
   a self-supporting waterproof substrate having first and second major surfaces;
   an adhesive layer disposed on at least a portion of the first major surface;
   a coating comprising the composition of any one of Embodiments S through W disposed on a least a portion of the adhesive layer; and
   a dry, cold-water soluble gelling agent positioned for fluidic contact with the nutrient medium.
Embodiment DD is the thin film culture device of Embodiment CC, further comprising a cover sheet coupled to a least a portion of the substrate, the cover sheet having a first side facing the first major surface.
Embodiment EE is the thin film culture device of Embodiment DD, wherein the gelling agent is adhered to the first side of the cover sheet.

Objects and advantages of this invention are further illustrated by the following examples, but the particular materials and amounts thereof recited in these examples, as well as other conditions and details, should not be construed to unduly limit this invention.

### EXAMPLES

**Table 1. Materials**

| Component | | Source |
|---|---|---|
| Buffered Peptone Water (ISO) (BPW (ISO)), powder formulation | Used as the starting material in Examples 1-3 and Example 8. Also used as a comparative example. | 3M Company. (St. Paul, MN) Catalog No. BPW500 |
| Buffered Peptone Water (ISO) (BPW (ISO)), granulated formulation | Used as a comparative example | EMD Millipore (Billerica, MA) Product No. 1.07228.0500 |
| Tryptic Soy Broth (TSB), powder formulation | Used as the starting material in Example 4 and used as a comparative example | Becton, Dickinson Co. (Franklin Lakes, NJ) Catalog No. 211824 |
| Tryptic Soy Broth (TSB), granulated formulation | Used as a comparative example | EMD Millipore (Billerica, MA) Product No. 1.05459.0500 |
| Modified Listeria Recovery Broth (mLRB), powder formulation | Used as the starting material in Example 5 and used as a comparative example | 3M Company (St. Paul, MN) Catalog No. MLRB500 |

### Test Methods

### Particle Size Determination for Agglomerated Samples:

For Examples 1-6 and Example 9, the agglomerated sample was passed through screens of 20, 40, 60, and 140 mesh. For each screening procedure, the fraction of material that failed to pass through the screen was collected and weighed.

### Loose Bulk Density:

The sample material was poured into a 50 mL graduated cylinder to the 20 mL mark. The net weight of the added material was recorded and loose bulk density was determined as the ratio of the sample weight to sample volume (g/mL). Duplicate measurements were taken for each sample and the bulk density was recorded as the mean value.

### Dissolution Time:

For BPW (ISO) samples, the solid material (2.55 g) was added in a single portion to a glass jar (diameter of 4 cm) containing 100 mL of deionized water maintained at room temperature. No agitation was provided to the liquid. The time required for the entire sample to dissolve, as determined by visual inspection, was recorded as the dissolution time. Duplicate measurements were taken for each sample and the dissolution time was recorded as the mean value.

The same procedure was used with TSB and mLRB agglomerated samples with the exception that, for TSB, 3.4 g of sample was used and, for mLRB, 4.4 g of sample were used.

### Moisture Content:

Samples were analyzed for moisture content (% water) by Karl Fischer analysis. Test samples were prepared by adding 0.1 g of sample material to a vial containing 10 mL of anhydrous methanol (Avantor Performance Materials, Center Valley, PA). The vial was sealed and stored in a dry box (under a nitrogen atmosphere) overnight. Duplicate vials were prepared for each sample. An aliquot of the extract (0.1 to 0.3 g) was removed from each vial and injected into a model 756 KF Coulometer (Metrohm AG, Herisau, Switzerland). Aquastar® Coulomat A (EMD Millipore, Billerica, MA) was used as the analyte solution. The measurements of moisture content were made in triplicate for each vial. As duplicate test samples were prepared, the total number of measurements made for each sample was six. The test method was standardized using NIST SRM 2890 certified Hydranal® Water Standard 1.00 (1.001 +/- 0.003 mg/g, Sigma-Aldrich Corporation, St. Louis, MO).

### Example 1

Buffered Peptone Water (ISO) powder (50 g) was loaded into the agglomerating chamber of a VFC-Lab Micro Flo-Coater® (Freund-Vector Corporation, Marion, IA) that had been preheated using an inlet air temperature of 65° C with an air flow setting of 130 liters per minute (LPM). The spray port was mounted on the top of the fluid bed system and deionized water was used as the agglomeration liquid. The agglomeration was conducted using the following instrument settings: air flow = 120 LPM; atomizer nozzle air pressure = 265 mbar; initial inlet temperature = 65° C; initial exhaust temperature = 41° C; filter pulse = 1 second; initial pump speed = 5 rpm. The inlet temperature and pump speed were adjusted during the agglomeration process to maintain an exhaust temperature of 39-42 ° C. The deionized water spray was stopped after 30.1 g of the deionized water was used. The resulting agglomerated sample was dried in the fluid bed until the exhaust temperature reached 45 ° C (about 5-10 minutes). The dried product was then sieved through screens to determine the particle size distribution (Table 2). The material collected between 20-140 mesh was tested for loose bulk density and dissolution time (Table 3).

**Table 2. Particle Size Distribution for BPW (ISO) Agglomerated (Example 1)**

| | Particle Size Distribution (%) | | | | |
|---|---|---|---|---|---|
| | <20 mesh (>841µm) | 20-40 mesh (841-400µm) | 40-60 mesh (400-250µm) | 60-140 mesh (250-105µm) | >140 mesh (<105 µm) |
| Example 1 | 3.3 % | 6.3 % | 43.1 % | 45.6 % | 1.7 % |

**Table 3. Comparison of BPW (ISO) Samples**

| Sample | Loose Bulk Density (g/mL) | Dissolution Time |
|---|---|---|
| BPW (ISO) Agglomerated (20-140 mesh) (Example 1) | 0.35 | 30 seconds |
| BPW (ISO) Powder (source: 3M Corporation) | 0.81 | >10 minutes |
| BPW (ISO) Granulated (source: EMD Millipore) | 0.81 | >20 minutes |

### Example 2

Buffered Peptone Water (ISO) powder (50 g) was loaded into the agglomerating chamber of a VFC-Lab Micro Flo-Coater® (Freund-Vector Corporation, Marion, IA) that had been preheated using an inlet temperature of 65 ° C with an air flow setting of 130 liters per minute (LPM). The spray port was mounted on the top of the fluid bed system. A 5% (weight to volume) solution of BPW in deionized water was used as the agglomeration liquid. The agglomeration was conducted using the following instrument settings: air flow = 131 LPM; atomizer nozzle air pressure = 315 mbar; initial inlet temperature = 65 ° C; initial exhaust temperature = 41 ° C; filter pulse = 1 second; initial pump speed = 5 rpm. The inlet temperature and pump speed were adjusted during the agglomeration process to maintain an exhaust temperature of 39-42 ° C. The spray was stopped after 20.0 g of the 5% BPW solution was used. The resulting agglomerated sample was dried in the fluid bed until the exhaust temperature reached 45 ° C (about 5-10 minutes). The dried product was then sieved through screens to determine the particle size distribution (Table 4). The material collected between 20-140 mesh was tested for loose bulk density (Table 5).

**Table 4. Particle Size Distribution for BPW (ISO) Agglomerated (Example 2)**

| | Particle Size Distribution (%) | | | | |
|---|---|---|---|---|---|
| | <20 mesh (>841 µm) | 20-40 mesh (841-400µm) | 40-60 mesh (400-250µm) | 60-140 mesh (250-105µm) | >140 mesh (<105 µm) |
| Example 2 | 0.8 % | 17.4 % | 48.2 % | 30.8 % | 2.8 % |

**Table 5. Comparison of BPW (ISO) Samples**

| Sample | Loose Bulk Density (g/mL) |
|---|---|
| BPW (ISO) Agglomerated (20-140 mesh) (Example 2) | 0.32 |
| BPW (ISO) Powder (source: 3M Corporation) | 0.81 |
| BPW (ISO) Granulated (source: EMD Millipore) | 0.81 |

### Example 3

The same procedure as described in Example 2 was followed with the exception that a 7.5% (weight to volume) solution of BPW in deionized water was used as the agglomeration liquid, instead of a 5% solution. The particle size distribution is reported in Table 6. The material collected between 20-140 mesh was tested for loose bulk density (Table 7).

**Table 6. Particle Size Distribution for BPW (ISO) Agglomerated (Example 3)**

| | Particle Size Distribution (%) | | | | |
|---|---|---|---|---|---|
| | <20 mesh (>841 µm) | 20-40 mesh (841-400µm) | 40-60 mesh (400-250µm) | 60-140 mesh (250-105µm) | >140 mesh (<105 µm) |
| Example 3 | 1.7% | 36.0 % | 42.3 % | 15.6 % | 4.4 % |

**Table 7. Comparison of BPW (ISO) Samples**

| Sample | Loose Bulk Density (g/mL) |
|---|---|
| BPW (ISO) Agglomerated (20-140 mesh) (Example 3) | 0.30 |
| BPW (ISO) Powder (source: 3M Corporation) | 0.81 |
| BPW (ISO) Granulated (source: EMD Millipore) | 0.81 |

### Example 4

Tryptic Soy Broth powder (50 g) was loaded into the agglomerating chamber of a VFC-Lab Micro Flo-Coater® (Freund-Vector Corporation, Marion, IA) that had been preheated using an inlet temperature of 65 °C with an air flow setting of 130 liters per minute (LPM). The spray port was mounted on the top of the fluid bed system and deionized water was used as the agglomeration liquid. The agglomeration was conducted using the following instrument settings: air flow = 170 LPM; atomizer nozzle air pressure = 250 mbar; initial inlet temperature = 65 °C; initial exhaust temperature = 41 °C; filter pulse = 1 second; initial pump speed = 5 rpm. The inlet temperature and pump speed were adjusted during the agglomeration process to maintain an exhaust temperature of 39-42 °C. The deionized water spray was stopped after 30.0 g of the deionized water was used. The resulting agglomerated sample was dried in the fluid bed until the exhaust temperature reached 45 ° C (about 5-10 minutes). The dried product was then sieved through screens to determine the particle size distribution (Table 8). The material collected between 20-140 mesh was tested for loose bulk density and dissolution time (Table 9).

**Table 8. Particle Size Distribution for TSB Agglomerated (Example 4)**

| | Particle Size Distribution (%) | | | | |
|---|---|---|---|---|---|
| | <20 mesh (>841µm) | 20-40 mesh (841-400µm) | 40-60 mesh (400-250µm) | 60-140 mesh (250-105µm) | >140 mesh (<105 µm) |
| Example 4 | 2.0 % | 25.6 % | 52.4 % | 19.0 % | 1.0 % |

**Table 9. Comparison of TSB Samples**

| Sample | Loose Bulk Density (g/mL) | Dissolution Time |
|---|---|---|
| TSB Agglomerated (20-140 mesh) (Example 4) | 0.26 | 30 seconds |
| TSB Powder (source: Becton, Dickinson Company) | 0.60 | 15 minutes |
| TSB Granulated (source: EMD Millipore) | 0.75 | >20 minutes |

### Example 5

Modified Listeria Recovery Broth powder (50 g) was loaded into the agglomerating chamber of a VFC-Lab Micro Flo-Coater® (Freund-Vector Corporation, Marion, IA) that had been preheated using an inlet temperature of 65 °C with an air flow setting of 130 liters per minute (LPM). The spray port was mounted on the top of the fluid bed system and deionized water was used as the agglomeration liquid. The agglomeration was conducted using the following instrument settings: air flow = 190 LPM; atomizer nozzle air pressure = 250 mbar; initial inlet temperature = 65 ° C; initial exhaust temperature = 43 °C; filter pulse = 1 second; initial pump speed = 5 rpm. The inlet temperature and pump speed were adjusted during the agglomeration process to maintain an exhaust temperature of 40-43 ° C. The deionized water spray was stopped after 30.0 g of the deionized water was used. The resulting agglomerated sample was dried in the fluid bed until the exhaust temperature reached 45 ° C (about 5-10 minutes). The dried product was then sieved through screens to determine the particle size distribution (Table 10). The material collected between 20-140 mesh was tested for loose bulk density and dissolution time (Table 11).

**Table 10. Particle Size Distribution for mLRB Agglomerated (Example 5)**

| | Particle Size Distribution (%) | | | | |
|---|---|---|---|---|---|
| | <20 mesh (>841µm) | 20-40 mesh (841-400µm) | 40-60 mesh (400-250µm) | 60-140 mesh (250-105µm) | >140 mesh (< 105 µm) |
| Example 5 | 0.8 % | 16.4 % | 44.4 % | 37.7 % | 0.7 % |

**Table 11. Comparison of mLRB Agglomerate to mLRB Powder**

| Sample | Loose Bulk Density (g/mL) | Dissolution Time |
|---|---|---|
| mLRB Agglomerated (20-140 mesh) (Example 5) | 0.38 | 60 seconds |
| mLRB Powder (source: 3M Corporation) | 0.55 | 15 minutes |

### Example 6

### The agglomerated BPW (ISO) product from Example 1 (2.55 g of 20-140 mesh sample) was quickly added in a single portion to a 150 mL glass beaker (diameter of 6 cm) containing 100 mL of deionized water maintained at room temperature. After standing for 30 seconds without any agitation, the contents of the beaker were vacuum filtered through Whatman™ #54 filter paper (55 mm diameter) using a Buchner funnel. The recovered solid material was dried in an oven (60 °C) for three hours and then weighed.

The same procedure was conducted using powdered BPW (ISO) and granulated BPW (ISO). The dissolution efficiency results comparing the agglomerated BPW (ISO) product of Example 1 with powdered BPW (ISO) and granulated BPW (ISO) are reported in Table 12.

**Table 12. Dissolution Efficiency of BPW Samples**

| Sample | Amount of Undissolved BPW (ISO) Recovered (g) | Percentage of Undissolved BPW (ISO) Recovered |
|---|---|---|
| BPW (ISO) Agglomerated (20-140 mesh) (Example 1) | 0 | 0% |
| BPW (ISO) Powder (source: 3M Corporation) | 0.88 | 35% |
| BPW (ISO) Granulated (source: EMD Millipore) | 1.45 | 57% |

### Example 7

The agglomerated Tryptic Soy Broth (TSB) product from Example 5 (1.5 g of 20-140 mesh sample) was quickly added in a single portion to a 150 mL glass beaker (diameter of 6 cm) containing 50 mL of deionized water maintained at room temperature. After standing for 30 seconds without any agitation, the contents of the beaker were vacuum filtered through Whatman™ #54 filter paper (55 mm diameter) using a Buchner funnel. The recovered solid material was dried in an oven (60 °C) for three hours and then weighed.

The same procedure was conducted using granulated TSB and powdered TSB. The dissolution efficiency results comparing the agglomerated TSB product of Example 4 with powdered TSB and granulated TSB are reported in Table 13.

**Table 13. Dissolution Efficiency of TSB Samples**

| Sample | Amount of Undissolved TSB Recovered (g) | Percentage of Undissolved TSB Recovered |
|---|---|---|
| TSB Agglomerated (20-140 mesh) (Example 4) | 0 | 0% |
| TSB Powder (source: Becton, Dickinson Co.) | 0.31 | 21% |
| TSB Granulated (source: EMD Millipore) | 0.80 | 53% |

### Example 8

Buffered Peptone Water (ISO) powder (1 Kg) was loaded into the agglomeration chamber of a Vector FL-M-1 Flo-Coater® (Freund-Vector Corporation, Marion, IA) that had been preheated using an inlet temperature of 70 °C with an air flow setting of 62 LPM. In addition to the standard bottom screen, a second fine mesh Dutch weave screen was used in order to prevent the powder from crossing the fluid bed. The spray port was mounted on the top of the fluid bed system and deionized water was used as the agglomeration liquid. The agglomeration was conducted using the following instrument settings: air flow = 108 LPM; atomizer nozzle pressure = 827 mbar; initial inlet temperature = 70 °C; initial exhaust temperature = 41 °C; initial pump speed = 8-10 rpm; filter pulse = 10-20 seconds. During the agglomeration process, the exhaust temperature was monitored and maintained at 39-42 ° C by adjusting the inlet temperature and the pump speed. The deionized water spray was stopped after 450 g of deionized water was used. The resulting agglomerated sample was dried in the fluid bed until the exhaust temperature reached 43 °C (about 5 minutes). The loose bulk density of the dried agglomerated product was 0.40 g/mL. The dried agglomerated product was then sieved through screens to determine the particle size distribution (Table 14). The dissolution times for the agglomerated product, as well as various fractions prepared through sieving were determined using the test method described above. The results are presented in Table 15.

**Table 14. Particle Size Distribution BPW (ISO) Agglomerated (Example 8)**

| | Particle Size Distribution (%) | | | | |
|---|---|---|---|---|---|
| | <20 mesh (>841µm) | 20-40 mesh (841-400µm) | 40-60 mesh (400-250µm) | 60-140 mesh (250-105µm) | >140 mesh (<105 µm) |
| Example 8 | 0 % | 1.9 % | 39.4 % | 54.0 % | 4.7 % |

**Table 15. Dissolution Times For Agglomerated Product from Example 8**

| Sample (Particle Size) | Dissolution Time |
|---|---|
| BPW (ISO) Agglomerated (20 to >140 mesh) (No sieving of sample) | 30 seconds |
| BPW (ISO) Agglomerated (20 to 40 mesh fraction) | 90 to 120 seconds |
| BPW (ISO) Agglomerated (40 to 60 mesh fraction) | 30 seconds |
| BPW (ISO) Agglomerated (60 to 140 mesh fraction) | 15 seconds |
| BPW (ISO) Agglomerated (>140 mesh fraction) | 90 seconds |
| BPW (ISO) Powder (source: 3M Corporation) | >10 minutes |

### Example 9

The mean moisture content (% water) of each of the following materials was determined using the test method described above: the agglomerated BPW (ISO) material (from Examples 1, 2 and 8); the agglomerated TSB material (from Example 4); the BPW (ISO) powder (sourced from the 3M Corporation); and the TSB powder (sourced from the Becton, Dickinson Company). The results are presented in Table 16.

**Table 16. Moisture Content**

| Sample | Mean Moisture Content (n=6) (% water) | Standard Deviation |
|---|---|---|
| BPW (ISO) Agglomerated (Example 1) | 2.94 % | 0.11 % |
| BPW (ISO) Agglomerated (Example 2) | 4.01 % | 0.15 % |
| BPW (ISO) Agglomerated (Example 8) | 2.50 % | 0.11 % |
| BPW (ISO) Powder (source: 3M Corporation) | 2.04 % | 0.15 % |
| TSB Agglomerated (Example 4) | 4.77 % | 0.05 % |
| TSB Powder (source: Becton, Dickinson Co.) | 5.16 % | 0.14 % |

### Example 10

Culture media broth was prepared by diluting the agglomerated BPW (ISO) product of Example 1 (2.56 g of the 20-140 mesh sample) in distilled water (100 mL). The resulting solution was sterilized by filtration through a 0.2 micron Supor® syringe filter (Pall Life Sciences, Ann Arbor, MI). The pH of the solution was measured to be approximately 7.0 (Beckman Φ350 pH Meter). Bacterial strains *E.coli* 0157:H7 (ATCC 700728), *E. coli* (ATCC 25922), *S*. *enteriditis* (ATCC13076), *S*. *typhi* (ATCC14028), and *P*. *aeruginosa* (ATCC 27853) were obtained from the American Type Culture Collection, Manassas, VA. A bacterial suspension was prepared by inoculating a pure culture of the microorganism into tryptic soy broth (Becton, Dickinson Co., Franklin Lakes, NJ) and incubating at 37 ° C overnight. Each of the individual bacterial strain suspensions was then diluted with Butterfield's Diluent (3M Company, St. Paul, MN) to a concentration of about 10⁵ cfu/mL.

Using a Falcon™ Clear 96-Well Microtest™ Plate (Becton, Dickinson Co.), 25 microliters of an individual bacterial dilution (105 cfu/mL) was spiked into 225 microliters of culture media broth (four replicates for each bacterial strain). The microtiter plate was incubated at 37 ° C for 24 hours in a PowerWave™ Microplate Reader (BioTek Corporation, Winooski, VT). The growth rate for each bacterial strain was determined by measuring the absorbance (at 640 nm) at 1 hour intervals. The microtiter plate was shaken for 5 seconds prior to each measurement. The absorbance readings for each of the bacterial strains are reported in Tables 17-21.

For comparison purposes, a similar growth study for each of the bacterial strains was conducted using the same procedure as above with the exception that BPW (ISO) powder (source 3M Corporation) was used to prepare the culture media broth, instead of agglomerated BPW (ISO). The results of these comparison studies are also shown in Tables 17-21. The data indicate that each organism grew approximately as fast in reconstituted agglomerated BPW medium as it did in reconstituted powdered BPW.

**Table 17. Growth Rate for E. coli 0157:H7 (ATCC 700728)**

| Culture Media Broth | Absorbance Measurement (640 nm) at Selected Incubation Time Points | | | | | | | | |
|---|---|---|---|---|---|---|---|---|---|
| | 0 hr | 1 hr | 3 hr | 5 hr | 7 hr | 10 hr | 15 hr | 20 hr | 24 hr |
| Broth prepared using Agglomerated BPW (ISO) | 0.07 | 0.07 | 0.22 | 0.51 | 0.55 | 0.61 | 0.62 | 0.64 | 0.64 |
| Broth prepared using powdered BPW (ISO) | 0.07 | 0.07 | 0.23 | 0.52 | 0.56 | 0.65 | 0.69 | 0.69 | 0.67 |

**Table 18. Growth Rate for E. coli (ATCC 25922)**

| Culture Media Broth | Absorbance Measurement (640 nm) at Selected Incubation Time Points | | | | | | | | |
|---|---|---|---|---|---|---|---|---|---|
| | 0 hr | 1 hr | 3 hr | 5 hr | 7 hr | 10 hr | 15 hr | 20 hr | 24 hr |
| Broth prepared using agglomerated BPW (ISO) | 0.07 | 0.07 | 0.19 | 0.51 | 0.54 | 0.52 | 0.51 | 0.53 | 0.53 |
| Broth prepared using powdered BPW (ISO) | 0.07 | 0.07 | 0.18 | 0.54 | 0.57 | 0.56 | 0.55 | 0.57 | 0.58 |

**Table 19. Growth Rate for S. enteriditis (ATCC 13076)**

| Culture Media Broth | Absorbance Measurement (640 nm) at Selected Incubation Time Points | | | | | | | | |
|---|---|---|---|---|---|---|---|---|---|
| | 0 hr | 1 hr | 3 hr | 5 hr | 7 hr | 10 hr | 15 hr | 20 hr | 24 hr |
| Broth prepared using agglomerated BPW (ISO) | 0.09 | 0.09 | 0.13 | 0.39 | 0.46 | 0.54 | 0.65 | 0.70 | 0.71 |
| Broth prepared using powdered BPW (ISO) | 0.09 | 0.09 | 0.13 | 0.40 | 0.46 | 0.56 | 0.64 | 0.69 | 0.70 |

**Table 20. Growth Rate for S. typhi (ATCC 14028)**

| Culture Media Broth | Absorbance Measurement (640 nm) at Selected Incubation Time Points | | | | | | | | |
|---|---|---|---|---|---|---|---|---|---|
| | 0 hr | 1 hr | 3 hr | 5 hr | 7 hr | 10 hr | 15 hr | 20 hr | 24 hr |
| Broth prepared using agglomerated BPW (ISO) | 0.08 | 0.09 | 0.18 | 0.34 | 0.52 | 0.63 | 0.74 | 0.78 | 0.79 |
| Broth prepared using powdered BPW (ISO) | 0.09 | 0.09 | 0.18 | 0.35 | 0.53 | 0.65 | 0.76 | 0.79 | 0.81 |

**Table 21. Growth Chart for P. aeruginosa (ATCC 27853)**

| Culture Media Broth | Absorbance Measurement (640 nm) at Selected Incubation Time Points | | | | | | | | |
|---|---|---|---|---|---|---|---|---|---|
| | 0 hr | 1 hr | 3 hr | 5 hr | 7 hr | 10 hr | 15 hr | 20 hr | 24 hr |
| Broth prepared using agglomerated BPW (ISO) | 0.09 | 0.09 | 0.21 | 0.51 | 0.57 | 0.63 | 0.68 | 0.71 | 0.73 |
| Broth prepared using powdered BPW (ISO) | 0.09 | 0.09 | 0.21 | 0.53 | 0.59 | 0.64 | 0.71 | 0.74 | 0.76 |

### Example 11

Agglomerated Buffered Peptone Water was prepared starting from the individual components. All of the components were in the powdered form (the powder was fine enough to pass through a 100 mesh sieve). Monopotassium phosphate (15 g), disodium hydrogen phosphate (30 g), sodium chloride (50 g), Peptone (100 g from Alpha Biosciences, Baltimore, MD), and the tracer dye phenol red (0.6 g from Sigma-Aldrich Corporation) were loaded into the agglomeration chamber of a VFC-Lab Micro Flo-Coater® (Freund-Vector Corporation, Marion, IA). The materials were blended at room temperature in the agglomeration chamber for 20 minutes with the air flow at 70 LPM. Following the blending step, the materials were agglomerated in a manner similar to the process described in Example 1. The unit was heated using an inlet temperature of 85° C with an air flow setting of 90 LPM. The spray port was mounted on the top of the fluid bed system and deionized water was used as the agglomeration liquid. The agglomeration was conducted using the following instrument settings: air flow = 90 LPM; atomizer nozzle pressure = 265 mbar; initial inlet temperature = 85° C; initial exhaust temperature = 45° C; initial pump speed = 16 rpm; filter pulse = 1 second. During the agglomeration process, the exhaust temperature was monitored and maintained at 42-45° C by adjusting the inlet temperature and the pump speed. The deionized water spray was stopped after 50 g of deionized water was used. The resulting agglomerated sample was dried in the fluid bed until the exhaust temperature reached 45° C (about 5-10 minutes). The agglomerated product was evaluated for uniform distribution of the individual components in the agglomerated material. Three samples (1 g) were randomly selected from the bulk material. Each sample was dissolved in water to a concentration of 0.1 g/mL and the absorbance value was measured at 550 nm using a UV-visible spectrophotometer. The mean absorbance measurement for the three samples was 0.520 with a standard deviation of 0.009, indicating uniform distribution of the individual components in the bulk product.

### Example 12

A test method based on sample absorbance was used to determine the dissolution time for agglomerated product prepared by the agglomeration method of Example 1, as well as the various fractions that were prepared through sieving. A 100 mL beaker containing 50 mL of deionized water (23° C water temperature) was placed on the platform of a manual lab jack (variable height adjustment in the vertical direction). An IKA RW 20 Digital Overhead Stirrer (IKA Works Inc., Wilmington, NC) with a 16.5 inch stainless steel basket shaft (catalog number 11010-006, VWR Scientific, Radnor, PA) was positioned above the beaker. The agglomerated material (2.0 g) was carefully added to a 100 mesh stainless steel basket (catalog number 89049-188, VWR Scientific) and the basket was attached to the end of the shaft using the spring style clip on the shaft. The stirring shaft was set to rotate at 450 rpm and the lab jack was quickly raised so that the entire mesh portion of the rotating basket was submerged in the water (ensuring submersion of the entire sample in the water). The recording of time began the instant that the basket contacted the water. Aliquots (500 microliters) were withdrawn from the beaker at the time points of 30, 60, 120, 180, 300, and 600 seconds. Each sample was dispensed into a well in a clear 96 well plate and the absorbance measurements were taken at 310 nm with a BioTek Synergy MX Microplate Reader (BioTek US, Winooski, VT). The results for dissolution are reported in Table 22. The absorbance at each time point was normalized with an absorbance value of 1.0 indicating the maximum measured absorbance for the sample.

The same procedure was also conducted using powdered BPW (ISO) and granulated BPW (ISO).

**Table 22.**

| Sample | Normalized Absorbance at Sampling Time Points (seconds) | | | | | |
|---|---|---|---|---|---|---|
| | 30 sec | 60 sec | 120 sec | 180 sec | 300 sec | 600 sec |
| Example 1 (unsieved) | 0.8 | 1.0 | 1.0 | 0.8 | 0.9 | 0.9 |
| Example 1 (< 20 mesh fraction) | 0.7 | 0.8 | 0.9 | 1.0 | 1.0 | 1.0 |
| Example 1 (20-40 mesh fraction) | 0.7 | 0.8 | 0.9 | 1.0 | 1.0 | 1.0 |
| Example 1 (40-60 mesh fraction) | 0.7 | 0.8 | 0.9 | 1.0 | 1.0 | 1.0 |
| Example 1 (60-100 mesh fraction) | 0.8 | 1.0 | 0.9 | 0.9 | 1.0 | 1.0 |
| Example 1 (100-140 mesh fraction) | 0.3 | 0.4 | 0.4 | 0.5 | 0.9 | 1.0 |
| Example 1 (>140 mesh fraction) | 0.3 | 0.3 | 0.4 | 0.6 | 1.0 | 1.0 |
| BPW (ISO) granulated formulation (source: EMD Millipore) | 0.3 | 0.5 | 0.8 | 1.0 | 0.9 | 0.9 |
| BPW (ISO) Powder Formulation (source: 3M Corporation) | 0.1 | 0.2 | 0.3 | 0.6 | 0.9 | 1.0 |

### Example 13

Agglomerated BPW (ISO) product (2.55 g of 20-140 mesh sample) prepared using the procedure of Example 1 was quickly added in a single portion to a 150 mL glass beaker (diameter of 6 cm) containing 100 mL of deionized water maintained at room temperature. After standing for 30 seconds without any agitation, the contents of the beaker were vacuum filtered using a Buchner funnel that was fitted with a pre-weighed sheet of Whatman™ #54 filter paper (55 mm diameter). The vacuum filtration step was completed in less than 5 seconds. The filter paper with recovered agglomerated BPW (ISO) product was carefully removed from the funnel, dried in an oven (80° C) for 15 hours, and then cooled to room temperature in a dessicator. The filter paper with dried product was weighed and the weight of the filter paper was subtracted from this value to provide the weight of the undissolved agglomerated BPW (ISO) product.

The same procedure was conducted with six additional sieved fractions of the agglomerated BPW (ISO) product (<20 mesh, 20-40 mesh, 40-60 mesh, 60-100 mesh, 100-140 mesh, and >140 mesh). The same procedure was also conducted using powdered BPW (ISO) and granulated BPW (ISO). The dissolution efficiency results comparing the agglomerated BPW (ISO) product with powdered BPW (ISO) and granulated BPW (ISO) are reported in Table 23.

**Table 23. Dissolution Efficiency of BPW Samples**

| Sample | Amount of Undissolved BPW (ISO) Recovered (g) | Percentage of Undissolved BPW (ISO) Recovered |
|---|---|---|
| BPW (ISO) Agglomerated (20-140 mesh | 0.012 | 0.5% |
| BPW (ISO) Agglomerated (< 20 mesh) | 0.418 | 16% |
| BPW (ISO) Agglomerated (20-40 mesh) | 0.040 | 2% |
| BPW (ISO) Agglomerated (40-60 mesh) | 0.001 | 0.04% |
| BPW (ISO) Agglomerated (60-100 mesh) | 0.001 | 0.04% |
| BPW (ISO) Agglomerated (100-140 mesh) | 0.004 | 0.2% |
| BPW (ISO) Agglomerated (>140 mesh) | 0.378 | 15% |
| BPW (ISO) Powder (source: 3M Corporation) | 0.725 | 28% |
| BPW (ISO) Granulated (source: EMD Millipore) | 1.56 | 61% |

The complete disclosure of all patents, patent applications, and publications, and electronically available material cited herein are incorporated by reference. In the event that any inconsistency exists between the disclosure of the present application and the disclosure(s) of any document incorporated herein by reference, the disclosure of the present application shall govern. The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

All headings are for the convenience of the reader and should not be used to limit the meaning of the text that follows the heading, unless so specified.

Various modifications may be made without departing from the spirit and scope of the invention. These and other embodiments are within the scope of the following claims.

### Claims of the parent application WO 2014/025514 A1

1. A method of making a flowable, agglomerated nutrient medium, the method comprising:
   forming a nutrient medium composition by:
      placing a powdered nutrient into a fluidized bed-type agglomeration chamber; wherein the powdered nutrient comprises a nutrient component that facilitates the growth of a microorganism;
      flowing a fluidizing gas through the chamber,
      contacting in the agglomeration chamber the powdered nutrient with an aerosol spray of an agglomeration liquid; and
   collecting the composition from the chamber;
   wherein the composition comprises a population of agglomerated nutrient medium particles and, optionally, nonagglomerated powdered nutrient;
   wherein the population comprises at least about 30 weight percent agglomerated nutrient particles having an effective particle diameter of about 250-400 microns.
2. The method of claim 1, wherein at least about 70 weight percent of particles in the population has an effective particle diameter of about 105-841 microns.
3. The method of claim 1 or claim 2, wherein less than 10 weight percent of particles in the population has an effective particle diameter of about 105 microns or smaller.
4. The method of any one of the preceding claims, further comprising isolating a subpopulation of agglomerated nutrient medium particles, the subpopulation having a predetermined effective particle diameter.
5. The method of any one of claims 1 through 4, wherein the agglomeration liquid comprises a solvent with a binder and/or a nutrient that facilitates the growth of a microorganism dissolved therein.
6. The method of any one of the preceding claims, wherein the powdered nutrient comprises a substantially uniform mixture of two or more powdered nutrients.
7. The article of any one of the preceding claims, wherein contacting the powdered nutrient with an aerosol spray of an agglomeration liquid comprises contacting the powdered nutrient with a predetermined volume of agglomeration liquid.
8. The method of any one of the preceding claims, wherein the fluidizing gas has a first temperature as it enters the agglomeration chamber, wherein the first temperature of the fluidizing gas is about 50° C to about 90° C.
9. The method of claim 8, wherein the fluidizing gas has a second temperature in a spraying zone of the agglomeration chamber, wherein the second temperature of the fluidizing gas is about 35° C to about 50° C.
10. The method of any one of the preceding claims, wherein the population of agglomerated nutrient medium particles has a mean water content of about 5.5 weight percent or less.
11. The method of any one of the preceding claims, wherein the population of agglomerated nutrient medium particles has a loose bulk density of about 0.2 to about 0.5 grams per cubic centimeter.
12. The method of any one of the preceding claims, wherein one or more powdered nutrient comprises a nutrient selected from the group consisting of a protein hydrolysate, a carbohydrate, a salt and a mixture of any two or more of the foregoing powdered nutrients.
13. The method of any one of the preceding claims, further comprising the step of subjecting the dried agglomerated nutrient medium to a process that reduces the number of viable microorganisms in the dried agglomerated nutrient medium.
14. The method of any one of the preceding claims, wherein placing a powdered nutrient into a fluidized bed-type agglomeration chamber comprises placing a plurality of powdered nutrients into the fluidized bed-type agglomeration chamber.
15. A composition comprising the flowable, dried agglomerated nutrient medium produced by the method of any one of the preceding claims.
16. A composition comprising an agglomerated nutrient medium comprising agglomerated particles that, when combined with an aqueous medium, form a mixture that facilitates the growth of a microorganism, wherein at least about 30 weight percent of the particles in the population has an effective particle diameter of about 250-400 microns, wherein the bulk particle density is about 0.2 to about 0.5 grams per cubic centimeter.
17. A capsule, a tablet, or a sachet comprising the composition of claim 15 or claim 16.
18. A kit comprising the capsule, tablet, sachet, and/or composition of any one of claims 15 through 17.
19. The kit of claim 18, wherein the composition is disposed in a package comprising a predetermined mass of the dried agglomerated nutrient medium, wherein the predetermined mass is selected to be mixed with 9.9 mL, 10 mL, 90 mL, 99 mL, 100 mL, 225 mL, 1.0 L, or 3.375 L of aqueous diluent to produce a reconstituted medium capable of facilitating the growth of a microorganism.
20. A thin film culture device, comprising:
   a self-supporting waterproof substrate having first and second major surfaces;
   an adhesive layer disposed on at least a portion of the first major surface;
   a coating comprising the composition of claim 15 or claim 16 disposed on a least a portion of the adhesive layer; and
   a dry, cold-water soluble gelling agent positioned for fluidic contact with the nutrient medium.

## Claims

1. A method of making a flowable, agglomerated nutrient medium, the method comprising:
forming a nutrient medium composition by:
placing a powdered nutrient into a fluidized bed-type agglomeration chamber; wherein the powdered nutrient comprises a nutrient component that facilitates the growth of a microorganism;
flowing a fluidizing gas through the chamber,
contacting in the agglomeration chamber the powdered nutrient with an aerosol spray of an agglomeration liquid; and
collecting the composition from the chamber;
wherein the composition comprises a population of agglomerated nutrient medium particles and, optionally, nonagglomerated powdered nutrient;
wherein the population of agglomerated nutrient medium particles after isolating the subpopulation has a loose bulk density of about 0.2 to about 0.5 grams per cubic centimeter.

2. The method of claim 1, wherein at least about 70 weight percent of particles in the population have an effective particle diameter of about 105-841 microns.

3. The method of claim 1 or claim 2, wherein less than 10 weight percent of particles in the population has an effective particle diameter of about 105 microns or smaller.

4. The method of any one of claims 1 through 3, wherein the agglomeration liquid comprises a solvent with a binder and/or a nutrient that facilitates the growth of a microorganism dissolved therein.

5. The method of any one of the preceding claims, wherein the fluidizing gas has a first temperature as it enters the agglomeration chamber, wherein the first temperature of the fluidizing gas is about 50° C to about 90° C.

6. The method of any one of the preceding claims, wherein the population of agglomerated nutrient medium particles after isolating the subpopulation has a mean water content of about 5.5 weight percent or less.

7. The method of any one of the preceding claims, wherein the powdered nutrient is buffered peptone water.

8. The method of claim 7, wherein the agglomeration liquid is a solution of buffered peptone water.

9. The method of any one of the preceding claims, wherein the population of agglomerated nutrient medium particles after isolating the subpopulation has a loose bulk density of about 0.26 to about 0.38 grams per cubic centimeter.

10. The method of any one of the preceding claims, wherein less than or equal to about 3.3 weight percent of the particles in the composition has an effective particle diameter greater than about 841 microns.

11. The method of any one of the preceding claims, wherein the nutrient medium is selected from modified listeria recovery broth or tryptic soy broth.

12. The method of any one of the preceding claims, the method further comprising isolating a subpopulation of agglomerated nutrient medium particles, the subpopulation having a predetermined effective particle diameter.

13. The method according to claim 12, wherein isolating a subpopulation comprises isolating a subpopulation having an effective particle diameter of 149 microns to 841 microns.

14. The method of any one of claims 1-13, wherein the population comprises at least about 30 weight percent agglomerated nutrient particles having an effective particle diameter of about 250-400 microns.

15. A composition comprising the flowable, dried agglomerated nutrient medium produced by the method of any one of the preceding claims.

16. A kit comprising the capsule, tablet, sachet, and/or composition of claim 15.
